(19) Europäisches Patentamt
European Patent Office
Office européen des brevets

(11) Veröffentlichungsnummer : **0 041 145**
**B1**

(12) # EUROPÄISCHE PATENTSCHRIFT

(45) Veröffentlichungstag der Patentschrift : **19.12.84**

(21) Anmeldenummer : **81103510.4**

(22) Anmeldetag : **08.05.81**

(51) Int. Cl.³ : **C 07 C127/19**, C 07 D207/06, C 07 D295/20, C 07 D265/30

(54) **Aralkylphenylharnstoffe und diese enthaltende Herbizide.**

(30) Priorität : **02.06.80 DE 3020869**

(43) Veröffentlichungstag der Anmeldung : **09.12.81 Patentblatt 81/49**

(45) Bekanntmachung des Hinweises auf die Patenterteilung : **19.12.84 Patentblatt 84/51**

(84) Benannte Vertragsstaaten : **AT BE CH DE FR GB IT LI NL SE**

(56) Entgegenhaltungen :
**EP-A- 0 040 859**
**CHEMICAL SENSES AND FLAVOUR, band 4, no. 1, 1979, DORDRECHT (NL) YAMOTO UND HASHIGAKI: " Chemical Structure and Sweet Taste of isocoumarins and related compounds ", Seiten 35-47**

(73) Patentinhaber : **BASF Aktiengesellschaft**
**Carl-Bosch-Strasse 38**
**D-6700 Ludwigshafen (DE)**

(72) Erfinder : **Schirmer, Ulrich, Dr.**
**Berghalde 79**
**D-6900 Heidelberg (DE)**
Erfinder : **Rohr, Wolfgang, Dr.**
**In der Dreispitz 13**
**D-6706 Wachenheim (DE)**
Erfinder : **Wuerzer, Bruno, Dr.**
**Ruedigerstrasse 13**
**D-6701 Otterstadt (DE)**

**EP 0 041 145 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue wertvolle Aralkylphenylharnstoffe mit herbizider Wirkung, Verfahren zur Herstellung dieser Aralkylphenylharnstoffe, Herbizide, welche diese Verbindungen enthalten, sowie Verfahren zur Bekämpfung unerwünschten Pflanzenwuchses mit diesen Verbindungen.

Es ist bekannt, daß Aralkoxyphenylharnstoffderivate z. B. der 1-(3-(2-Phenylethoxy)-phenyl)-3-methoxy-3-methylharnstoff herbizide Eigenschaften haben (DE-OS 28 46 723).

In der älteren Patentanmeldung EP-40859 werden Aralkylphenylharnstoffe mit herbizider Wirkung beschrieben. Die Literaturstelle CHEMICAL SENSES AND FLAVOUR, band 4, n° 1, 1979 DORDRECHT (NL) Seite 41, Tabelle V, n° LXXI, beschreibt einen Aralkylphenylharnstoff ohne einen Hinweis auf eine herbizide Wirkung.

Es wurde nun gefunden, daß Aralkylphenylharnstoffe der Formel

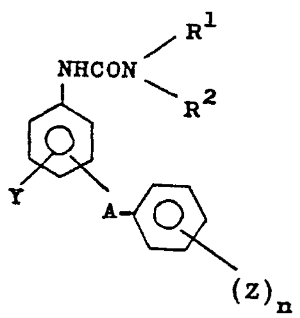

in der

$R^1$ und $R^2$ jeweils unabhängig voneinander Wasserstoff, Methoxy, Ethoxy oder ggf. durch Halogen, Methoxy, Ethoxy oder Cyano substituiertes Alkyl mit 1 bis 4 Kohlenstoffatomen, Cyclopropyl, Cyclohexyl, Allyl, Propargyl oder sekundäres Butinyl oder aber $R^1$ und $R^2$ zusammen eine ggf. durch Methyl oder Ethyl substituierte und/oder ggf. durch Sauerstoff oder Schwefel unterbrochene Alkylenkette mit 2 bis 8 Kohlenstoffatomen,

Y Wasserstoff, Methyl, Tertiärbutyl, Halogen, Methoxy, Ethoxy oder Trifluormethyl,

A einen ggf. durch Methyl oder Ethyl substituierten Alkylenrest mit 1 bis 10 Kohlenstoffatomen,

Z Wasserstoff, Methyl, Ethyl, Tertiärbutyl, Trifluormethyl, Methoxymethyl, Cyclohexyl, Benzyl, Phenyl, Phenoxy, Halogen, eine $C_4H_4$-Kette, die mit dem Benzolring zu einem ggf. substituierten Naphthylring kondensiert ist, Methoxy, Ethoxy, Isopropoxy, Hexyloxy, Tetrafluorethoxy, Methylthio, Thiocyanato, Cyano,

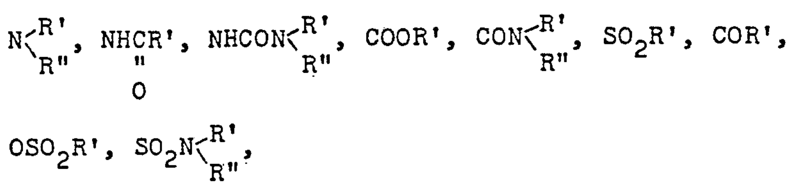

bedeutet, wobei R′ und R″ jeweils unabhängig voneinander Wasserstoff, Methyl, Ethyl, Methoxy, tert.-Butoxy, Methylthio, Cyclohexyl oder ggf. durch Methyl, Ethyl, Halogen, Methoxy oder Ethoxy substituiertes Phenyl bedeuten und n die Zahlen 1 bis 4 bedeutet, ausgenommen diejenigen Verbindungen, in denen A $(CH_2)_2$ und gleichzeitig $R^1$ und $R^2$ Wasserstoff und Z Wasserstoff bedeutet, sowie derjenigen Verbindung, in denen A $(CH_2)_3$ oder $(CH_2)_4$ und gleichzeitig $R^1$ Methyl, $R^2$ Methyl oder Methoxy, Y Wasserstoff oder Chlor, Z Wasserstoff oder $C_1$-$C_4$-Alkyl und n = 1 bedeutet, eine gute herbizide Wirkung bei zahlreichen wichtigen unerwünschten Pflanzen haben und eine gute Verträglichkeit bei verschiedenen Kulturpflanzen zeigen.

Die in der allgemeinen Formel angeführten Reste können beispielsweise folgende Bedeutungen haben:

$R^1$ und $R^2$ jeweils unabhängig voneinander Wasserstoff, Methoxy, Ethoxy, unsubstituiertes Alkyl mit 1 bis 4 C-Atomen durch Halogen oder Methoxy, Ethoxy oder Cyan substituiertes Alkyl mit 1 bis 4 C-Atomen, Methyl, Ethyl, Isopropyl, n-Propyl, n-Butyl, sec.-Butyl, tert.-Butyl, iso-Butyl, Cyclopropyl, Cyclohexyl, 2-Chorethyl, 2-Methoxyethyl, 2-Cyanomethyl, sec.-Butinyl, Propargyl, Allyl oder aber $R^1$ und $R^2$ zusammen eine gegebenenfalls durch Alkyl substituierte und/oder durch Sauerstoff oder Schwefel unterbrochene Alkylenkette mit 2 bis 8 Kohlenstoffatomen (z. B. Propylen, Butylen, Pentylen, Hexylen, 1,4-Dimethyl-butylen, 3-Oxapentylen, 3-Thiapentylen), Y Wasserstoff, Methyl, t-Butyl, Halogen (z. B. Fluor, Chlor, Brom), Methoxy oder Trifluormethyl,

A einen gegebenenfalls durch Alkyl substituierten Alkylenrest mit 1 bis 10 Kohlenstoffatomen (z. B. Methylen, Methylmethylen, Dimethylmethylen, Propylen, Hexylen, Ethylen, Methylethylen, Methylpropylen, Ethylpropylen, Butylen, Pentylen, Methylpentylen, Dimethylpropylen, Heptylen, Ethylbutylen, Trimethylpentylen),

Z Wasserstoff, Methyl, Ethyl, t-Butyl, Trifluormethyl, Methoxymethyl, Cyclohexyl, Benzyl, Phenyl, Phenoxy, Halogen (z. B. Fluor, Chlor, Brom oder Jod), eine $C_4H_4$-Kette, die mit dem Benzolring zu einem gegebenenfalls substituierten Naphthylring kondensiert ist, Isopropoxy, Hexyloxy, 1,1,2,2-Tetrafluorethoxy, Methylthio, Thiocyanato, Cyano,

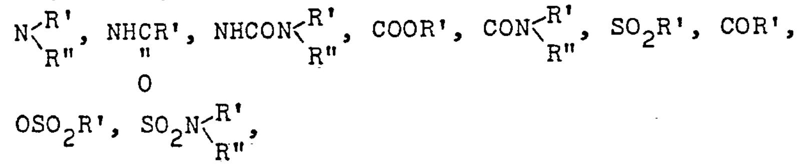

wobei R′ und R″ jeweils unabhängig voneinander Wasserstoff, Methyl, Ethyl, Methoxy, tert.-Butoxy, Methylthio, Cyclohexyl oder gegebenenfalls durch Methyl, Ethyl, Halogen oder Methoxy, Ethoxy subsitutiertes Phenyl (z. B. Phenyl, 3-Chlorphenyl, 4-Methylphenyl, 3-Methoxyphenyl) bedeuten kann.

Die neuen Verbindungen können beispielsweise nach folgendem Verfahren hergestellt werden :

Ein Aralkylphenylisocyanat der Formel

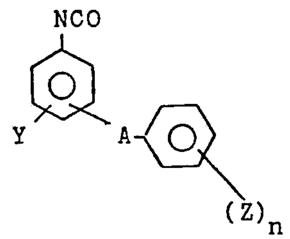

in der Y, A, $(Z)_n$ die oben genannten Bedeutungen haben, wird mit einem Amin der allgemeinen Formel

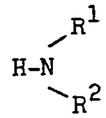

in dem $R^1$ und $R^2$ wie oben definiert ist, zur Reaktion gebracht.

Die Umsetzung der Aralkylphenylisocyanate erfolgt mit oder ohne einen für Isocyanatreaktionen gebräuchlichen Katalysatoren, z. B. tert. Amine (Triethylamin, 1,4-Diazabicyclo-(2,2,2)-octan), stickstoffhaltige Heterocyclen (Pyridin, 1,2-Dimethylimidazol) oder organische Zinnverbindungen (Dibutylzinndiacetat, Dimethylzinndichlorid) gegebenenfalls in einem unter den Reaktionsbedingungen inerten Lösungsmittel, z. B. Kohlenwasserstoff (Ligroin, Benzin, Toluol, Pentan, Cyclohexan), Halogenkohlenwasserstoff (Methylenchlorid, Chloroform, Dichlorethan, Chlorbenzol, o-, m- oder p-Dichlorbenzol), Nitrokohlenwasserstoff (Nitrobenzol, Nitromethan), Nitrile (Acetonitril, Butyronitril, Benzonitril), Ether (Diethylether, Tetrahydrofuran, Dioxan), Ester (Essigsäureethylester, Propionsäuremethylester) Ketone (Aceton, Methylethylketon) oder Amide (Dimethylformamid, Formamid) bei Temperaturen im Bereich von 0 bis 150 °C, vorzugsweise im Bereich von 40 bis 100 °C (S. Petersen in Methoden der Organ. Chemie, Band VIII, Seite 132, Georg-Thieme-Verlag, Stuttgart, 4. Auflage, (1952)).

Zur Herstellung der in diesem Verfahren benötigten Aralkylphenylisocyanate setzt man Aralkylaniline der Formel

nach bekannten Methoden mit Phosgen um (W. Siefken, J. Liebigs Annalen der Chemie *562,* 75 ff, (1949)).

Zur Herstellung dieser Aralkylaniline unterwirft man Nitrobenzole der allgemeinen Formel

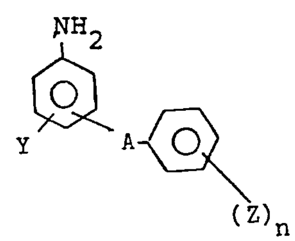

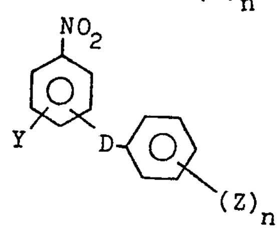

in der

D eine gesättigte oder ungesättigte, gegebenenfalls durch Alkyl substituierte Alkylenkette bedeutet, die gegebenenfalls durch eine Ketogruppe unterbrochen sein kann, oder D eine Ketogruppe bedeutet, Y und $(Z)_n$ die oben genannten Gruppen bedeuten,

der katalytischen Hydrierung, wobei man einen üblichen Hydrierkatalysator (z. B. Palladium auf Kohle, Platinschwamm oder Raney-Nickel), ein geeignetes Lösungsmittel wie Carbonsäure (z. B. Essigsäure), Ether (z. B. Tetrahydrofuran) oder Alkohole (z. B. Ethanol) und gegebenenfalls eine starke Säure (z. B. Methansulfonsäure, Schwefelsäure) bei Temperaturen zwischen 0 und 150 °C sowie Drücke zwischen 1 und 100 bar anwendet (B. R. Baker et al, J. Pharm. Sci. *56,* 737-742).

Die benötigten Nitrobenzole lassen sich nach bekannten Verfahren herstellen (z. B. B. R. Baker et al, J. Pharm. Sci. *56,* 737-742 (1967) ; P. Pfeiffer et al, J. Prakt. Chem. 109, 41 (1925) ; R. Geigy und W. Koenigs, Chem. Ber. *18,* 2401-2407, (1885) ; P. Petrenko-Kritschenko, Chem. Ber. *25,* 2239-2242, (1892).

Die folgenden Beispiele sollen die Herstellung der neuen Aralkylanilinderivate und ihrer Vorprodukte erläutern.

## Vorschrift A

3-(3-Phenylpropyl)-phenylisocyanat

In 200 ml Toluol werden bei − 10 °C 170 g Phosgen gelöst. Bei − 10 °C werden langsam 145 g 3-(3-Phenylpropyl)-anilin in 300 ml Toluol zugetropft. Man erwärmt langsam, leitet ab 60 °C Phosgen ein und bringt zum Sieden. Nach 6 Stunden wird überschüssiges Phosgen mit Stickstoff ausgeblasen und destilliert. Man erhält 156 g (3-(3-Phenylpropyl)-phenylisocyanat vom Siedepunkt 107-110 °C/0,1 mbar.

## Vorschrift B

3-(3-Phenylpropyl)-anilin

253 g 1-Phenyl-3-(3'-Nitrophenyl)-propen-2-on-1 werden in 2,5 l Eisessig suspendiert. Man gibt 196 g konz. Schwefelsäure sowie 10 g 10 % Pd auf Tierkohle zu und hydriert mit 1,1 bar Wasserstoffdruck bei 65-70 °C bis kein Wasserstoff mehr aufgenommen wird. Nach dem Abkühlen destilliert man einen Teil der Essigsäure ab, macht den Rückstand mit Natronlauge alkalisch, schüttelt mit Ether aus, trocknet die org. Phase mit Natriumsulfat, filtriert, zieht das Lösungsmittel ab und destilliert den Rückstand. So erhält man 150 g 3-(3-Phenylpropyl)-anilin vom Siedepunkt 148 °C/0,2 mbar.

Entsprechend können folgende Aralkylaniline hergestellt werden :

3-Benzylanilin (Fp 43-45 °C)
3-(3-(2-Chlorphenyl)-propyl)-anilin ($Kp_{0,1}$ 156-158)
3-(3-(4-Chlorphenyl)-propyl)-anilin ($Kp_{0,1}$ 160-161)
3-(3-(2-Methoxyphenyl)-propyl)-anilin ($Kp_{0,3}$ 168-170)
3-(3-(3-Methoxyphenyl)-propyl)-anilin ($Kp_{0,4}$ 181-182)
3-(3-(3-Methylphenyl)-propyl)-anilin ($Kp_{0,01}$ 142-145)
3-(3-(4-Methylphenyl)-propyl)-anilin ($Kp_{0,1}$ 148-150)
3-(3-(3-Trifluormethylphenyl)-propyl)-anilin ($Kp_{0,3}$ 142-145)
3-(3-(4-Fluorphenyl)-propyl)-anilin ($Kp_{0,1}$ 125-127)
3-(3-(4-tert.-Butylphenyl)-propyl)-anilin ($Kp_{0,4}$ 180-185)
3-(3-(4-Phenylphenyl)-propyl)-anilin (Fp. 76-77)
3-(3-(4-Ethylphenyl)-propyl)-anilin ($Kp_{0,2}$ 165-168)
3-(3-(3-Chlorphenyl)-propyl)-anilin ($Kp_{0,4}$ 165-172)
3-(3-(3-Hydroxyphenyl)-propyl)-anilin (Fp. 59-61 °C)
3-(3-(3,4-Dimethoxyphenyl)-propyl)-anilin ($Kp_{0,15}$ 195)
3-(3-(4-Methoxyphenyl)-propyl)-anlin ($Kp_{0,1}$ 176)
4-(3-Phenylpropyl)-anilin ($Kp_{0,4}$ 156-157)
3-(2-Methyl-3-phenyl-propyl)-anilin ($Kp_{0,4}$ 152-153)
3-(2-Methyl-3-(4-Fluorphenyl)-propyl)-anilin
3-(4-Methyl-5-phenyl-pentyl)-anilin ($Kp_{0,3}$ 177-179)
3-(2-Ethyl-3-phenyl-propyl)-anilin ($Kp_{0,2}$ 158-162)
4-(3-(4-methylphenyl)propyl)-anilin (Fp. 53-56 °C)
3-(3-phenylpropyl)-4-bromanilin ($Kp_{0,1}$ 159-160)
4-(3-(5-Fluorphenyl)-propyl)-anilin ($Kp_{0,1}$ 146-147)
4-(3-(2-Methylphenyl)-propyl)-anilin ($Kp_{0,1}$ 160-162)
4-(3-(3-Methylphenyl)-proipyl)-anilin
4-(4'-Phenyl)-benzylanilin (Fp. 105-107)
4-Benzylanilin (Fp. 35-37)
4-(3-(3-Methoxyphenyl)-propyl)-anilin ($Kp_{0,1}$ 178-180)

4-(2-Phenylethyl)-anilin (Fp. 42-45)
3-(2-Phenylethyl)-anilin (Fp. 50-52)
4-(4-Phenylbutyl)-anilin (Öl)
3-(4-Phenylbutyl)-anilin (Öl)
3-(3-(2-Methylphenyl)-propyl-anilin ($Kp_{0,2}$ 150-153)
3-(3-(3-Phenoxyphenyl)-propyl)-anilin ($Kp_{0,2}$ 195)
3-(3-(4-Ethoxyphenyl)-propyl)-anilin ($Kp_{0,3}$ 177)
3-(3-Tetrafluorethoxy)-phenyl)-anilin ($Kp_{0,1}$ 155)
3-(3-(4-Chlorphenyl)-propyl)-anilin ($Kp_{0,1}$ 150-154)
3-(3-(4-Dichlorphenyl)-propyl)-anilin ($Kp_{0,1}$ 177-182)
3-(3-(α-Naphthyl)-propyl)-anilin

Beispiel 1

Zu einer Mischung aus 23,7 g 3-(3-Phenylpropyl)-phenylisocyanat und 200 ml Toluol tropft man bei 0 °C 11 g 2,5-Dimethylpyrrolidin. Nach 6 stündigem Rühren wird die Mischung eingeengt und mit Petrolether verrührt. Man erhält 25,1 g weiße Kristalle mit einem Schmelzpunkt von 86-88 °C und folgender Strukturformel (Wirkstoff Nr. 1).

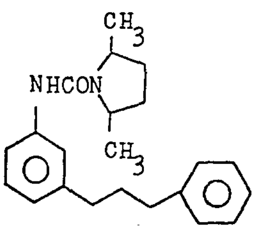

Beispiel 2

Zu einer Mischung aus 15 g 3-(3-(4-Chlorphenyl)-propyl)-anilin, 8 g $NaHCO_3$ und 200 ml THF tropft man bei 20 °C 7,6 g N-Methyl-N-methoxycarbamidsäurechlorid und läßt über Nacht rühren. Nach dem Filtrieren und Einengen der Lösung wird der Rückstand mit einer Mischung aus 90 Teilen Toluol und 10 Teilen Essigester über Kieselgel chromatographiert. Man erhält 14,6 g Produkt mit einem Schmelzpunkt von 73 bis 76 °C und folgender Strukturformel (Wirkstoff Nr. 2) :

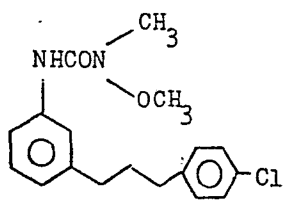

Beispiel 3

Eine Mischung aus 42,2 g 3-(3-Phenylpropyl)-anilin, 12,2 g Methylisocyanat, 2 Tropfen Dibutylzinndiacetat und 400 ml THF läßt man 4 Tage bei 20 °C stehen, dann engt man ein und verreibt den Rückstand mit Petrolether. Man erhält 53 g einer weißen Substanz vom Fp. 104-106 °C mit folgender Strukturformel (Wirkstoff Nr. 3) :

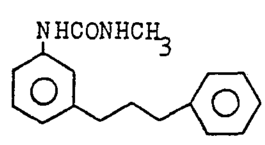

Nach einem dieser Verfahren können auch folgende Verbindungen hergestellt werden :

(Siehe Tabelle Seite 6 ff.)

Tabelle

| No. | 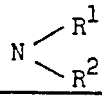 | Y | A | (Z)$_n$ | Fp °C |
|---|---|---|---|---|---|
| 4 | $NHCH_3$ | H | $3\text{-}CH_2$ | H | 153-154 |
| 5 | " | H | $3(CH_2)_2$ | H | |
| 6 | " | H | $4(CH_2)_3$ | H | 107-110 |
| 7 | " | 3Cl | $4(CH_2)_3$ | H | |
| 10 | NH cycl. $C_3H_5$ | H | $3(CH_2)_3$ | H | 113-115 |
| 11 | $N(C_2H_5)_2$ | H | " | H | 85- 87 |
| 12 | $N(C_2H_5)(nC_4H_9)$ | H | " | H | Öl |
| 13 | $NHC_2H_5$ | H | " | H | 83- 85 |
| 14 | 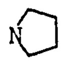 | H | " | H | 88- 90 |
| 15 | 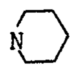 | H | " | H | 112-114 |
| 16 | 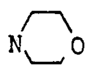 | H | " | H | 111-113 |
| 17 | $N(CH_3)$ cycl. $C_6H_{11}$ | H | " | H | 135-137 |
| 18 | $NHCH_2CH_2Cl$ | H | " | H | |
| 19 | NH iso $C_3H_7$ | H | " | H | |
| 20 | $NHCH_2CH_2OCH_3$ | H | " | H | |
| 21 | $NHCH_2CH_2CN$ | H | " | H | |
| 22 | $NHCH_3$ | H | " | $3OCH_3$ | 84- 85 |
| 23 | " | H | " | 4Cl | 147-148 |
| 24 | " | H | " | $3NHCONHCH_3$ | |
| 25 | " | $4OCH_3$ | " | 4SCN | |
| 26 | " | " | " | $4CH_2OCH_3$ | |
| 27 | " | $4CH_3$ | $3(CH_2)_3$ | 3CN | |
| 29 | " | H | $3(CH_2)_4$ | H | |
| 30 | " | " | " | $4CH_2C_6H_5$ | |
| 31 | " | 4Cl | $3(CH_2)_3$ | H | |
| 33 | $NHCH_3$ | H | " | 4-tert.$C_4H_9$ | 123-125 |
| 34 | " | " | " | 3Br | |
| 35 | " | " | " | $3OisoC_3H_7$ | |
| 36 | " | " | " | $3OC_2H_5$ | |
| 37 | " | " | " | $4OCH_3$ | |
| 38 | " | " | " | 4F | 129-130 |
| 40 | " | " | " | $4SCH_3$ | |
| 41 | " | " | " | $4N(CH_3)_2$ | |
| 42 | " | " | " | $3,4Cl_2$ | |
| 43 | " | " | " | $2,5Cl_2$ | |
| 44 | " | " | " | $2,4Cl_2$ | |
| 45 | " | " | " | $2,4(CH_3)_2$ | |
| 46 | " | " | " | $4C_6H_5$ | 156-158 |

Tabelle (Fortsetzung)

| No. | $N<^{R^1}_{R^2}$ | Y | A | $(Z)_n$ | Fp °C |
|---|---|---|---|---|---|
| 47 | " | " | " | $3CH_3$ | 104-105 |
| 48 | " | " | " | $4CH_3$ | 124-125 |
| 49 | " | " | " | 4Br | |
| 50 | " | " | $3(CH_2)_3$ | $3COOC_2H_5$ | |
| 51 | " | " | " | 3Cl | |
| 52 | $NHCH_3$ | H | $3(CH_2)_3$ | 2Cl | |
| 53 | " | " | " | $2CH_3$ | |
| 54 | " | " | " | $2OCH_3$ | |
| 55 | " | " | " | 2,3(CH=CH-CH=CH) | |
| 56 | " | " | " | $3CF_3$ | 125-126 |
| 57 | " | " | $3CH_2CH(CH_3)CH_2$ | H | 82- 84 |
| 58 | " | " | " | 4F | |
| 59 | " | " | $3(CH_2)_3CH(CH_3)CH_2$ | H | Öl |
| 60 | " | " | $3CH_2CH(C_2H_5)CH_2$ | H | Öl |
| 61 | $N(CH_3)_2$ | " | $3(CH_2)_3$ | 2Cl | |
| 62 | " | " | " | 3Cl | |
| 63 | " | " | " | 4Cl | |
| 64 | $N(CH_3)_2$ | H | $3(CH_2)_3$ | 2,4 $Cl_2$ | |
| 65 | " | " | " | 2,5 $Cl_2$ | |
| 66 | " | " | " | 3,4 $Cl_2$ | |
| 70 | " | " | " | 2,4 $(CH_3)_2$ | |
| 71 | " | " | " | 3,4 $(CH_3)_2$ | |
| 72 | " | " | " | 3 $OCH_3$ | |
| 73 | " | " | " | 4 $OCH_3$ | |
| 74 | " | " | " | $3OisoC_3H_7$ | |
| 76 | " | " | " | 4 $C_6H_5$ | |
| 77 | " | " | " | 3 $CF_3$ | |
| 78 | " | " | " | 4 Br | |
| 79 | $N(OCH_3)CH_3$ | " | " | 2 Cl | |
| 80 | " | " | " | 3 Cl | 46- 48 |
| 81 | " | " | " | 2,4 $Cl_2$ | |
| 82 | " | " | " | 3,4 $Cl_2$ | 54- 56 |
| 83 | " | " | " | 2,5 $Cl_2$ | |
| 87 | $N(OCH_3)CH_3$ | H | $3(CH_2)_3$ | 2,4 $(CH_3)_2$ | |
| 88 | " | " | " | 3,4 $(CH_2)_3$ | |
| 89 | " | " | " | 3 $OCH_3$ | 76- 77 |
| 90 | " | " | " | 4 $OCH_3$ | |
| 91 | " | " | " | 4 $C_6H_5$ | |
| 92 | " | " | " | 3 $CF_3$ | |
| 93 | " | " | " | 2,3(CH=CH-CH=CH) | 93- 95 |
| 97 | $NHCH_3$ | H | $3(CH_2)_3$ | 4 $C_2H_5$ | 109-111 |

Tabelle (Fortsetzung)

| No. | 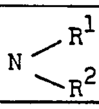 | Y | A | $(Z)_n$ | Fp °C |
|---|---|---|---|---|---|
| 99 | " | 4 Br | " | H | 156-158 |
| 100 | 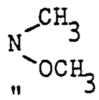 | " | $3-CH_2-CH_2-CH_2-$ | " | 72- 74 |
| 101 | " | H | " | $4-OC_2H_5$ | 55- 57 |
| 102 | " | " | " | $3-OCF_2CHF_2$ | Öl |
| 103 | " | " | " | 3,4-CH=CH-CH=CH- | |
| 104 | $NHCH_3$ | " | " | $4-OC_2H_5$ | 93- 95 |
| 105 | 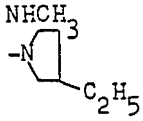 | " | " | H | 65- 67 |
| 106 | 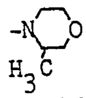 | " | " | " | 90- 92 |
| 107 | $-NHCH_3$ | " | $4-CH_2-CH_2-CH_2-$ | $4-CH_3$ | 139-142 |
| 109 | 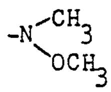 | " | $3-CH_2-CH_2-CH_2-$ | 3 O- 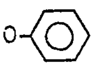 | Öl |
| 110 | 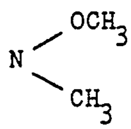 | H | 4 $CH_2CH_2CH_2$ | 4F | 92- 94 |
| 114 | " | " | " | 4Cl | 76- 77 |
| 115 | " | " | " | $4NHCOCH_3$ | |
| 116 | " | " | " | $4N(C_2H_5)_2$ | |
| 117 | " | " | " | $4N(CH_3)_2$ | 59- 61 |
| 118 | " | " | " | 4 $OC_6H_{13}$ | |
| 119 | " | " | " | 2Cl | |
| 120 | " | " | " | 3Cl | |
| 122 | " | " | " | 3-phenyl | |
| 123 | " | " | " | 4-phenyl | |
| 124 | " | " | " | 3 $OC_2H_5$ | |
| 127 | " | " | " | $2CF_3$ | |
| 128 | " | " | " | $3CF_3$ | |
| 129 | " | " | " | $4CF_3$ | |
| 130 | " | " | " | 2 $OCH_3$ | |
| 131 | " | " | " | 3 $OCH_3$ | 82- 84 |
| 132 | " | " | " | 4 $OCH_3$ | |
| 133 | " | " | " | $2,4,6(CH_3)_3$ | |
| 134 | " | " | " | $3,4Cl_2$ | |
| 136 | " | " | " | 3 $OSO_2CH_3$ | |
| 137 | 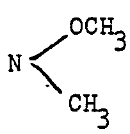 | H | 4 $CH_2CH_2CH_2$ | $2,4(CH_3)_2$ | |

Tabelle (Fortsetzung)

| No. | 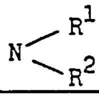 | Y | A | $(Z)_n$ | Fp °C |
|---|---|---|---|---|---|
| 139 | " | " | " | 2F | |
| 140 | " | " | " | 4 $OC_2H_5$ | |
| 141 | 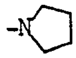 | " | " | H | |
| 142 | 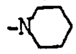 | " | " | H | |
| 143 | $-N(C_2H_5)_2$ | " | " | H | |
| 144 | 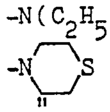 | " | " | H | |
| 145 | " | " | " | $4CH_3$ | |
| 146 | " | " | " | 4F | |
| 147 | " | " | " | 4Cl | |
| 148 | 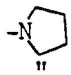 | " | " | $4CH_3$ | |
| 149 | " | " | " | 4F | |
| 150 | " | " | " | 4Cl | |
| 151 | $NHC_2H_5$ | " | " | $4CH_3$ | |
| 153 | 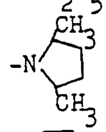 | " | " | " | |
| 154 | 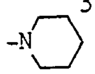 | " | " | " | |
| 155 | 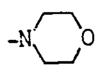 | " | " | " | |
| 156 | $N(C_2H_5)_2$ | " | " | " | |
| 157 | $NHOCH_3$ | " | " | " | |
| 158 | $NHOC_2H_5$ | " | " | " | |
| 159 | $NHCH_2CH=CH_2$ | " | " | " | |
| 160 | $N(CH_3)C_4H_9$ | H | 4 $CH_2CH_2CH_2$ | $4CH_3$ | |
| 161 | $NHC(CH_3)_2C{\equiv}CH$ | H | " | " | |
| 162 | $N(CH_3)_2$ | " | " | 4Cl | |
| 163 | " | " | " | 4F | |
| 164 | " | " | " | 4 $OCH_3$" | |
| 165 | $N(OCH_3)CH_3$ | " | 3 $CH_2CH_2CH_2$ | 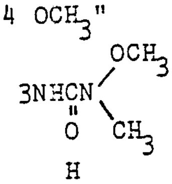 | 98-100 |
| 166 | " | " | 4 $CH_2$ | H | 86- 88 |
| 167 | " | " | " | 4-phenyl | 117-120 |
| 168 | " | $3CF_3$ | 4 $CH_2CH_2CH_2$ | H | |
| 169 | " | H | 4 $(CH_2)_5$ | H | |
| 170 | " | " | 4 $CH(CH_3)CH_2$ | H | |
| 171 | " | " | 4 $CH_2CH(CH_3)$ | H | |
| 172 | " | " | 4 $C(CH_3)_2$ | H | |
| 175 | " | " | 3 $(CH_2)_3CHCH_2$<br>$CH_3$ | H | Öl |

Tabelle (Fortsetzung)

| No. | 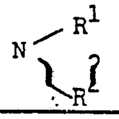 | Y | A | $(Z)_n$ | Fp °C |
|---|---|---|---|---|---|
| 176 | " | " | 4 $(CH_2)_3CHCH_2$ ($CH_3$) | H | |
| 177 | " | " | 4 $CH_2CHCH_2$ ($C_2H_5$) | H | |
| 178 | " | " | " | $4CH_3$ | |
| 179 | " | " | 3 $CH_2CHCH_2$ ($CH_3$) | 4F | Öl |
| 180 | " | " | 4 $CH_2CHCH_2$ ($CH_3$) | H | 54- 56 |
| 181 | " | " | " | $4CH_3$ | |
| 182 | 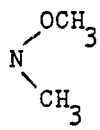 | H | 4 $CH_2CH-CH_2$ ($CH_3$) | 4Cl | |
| 184 | " | " | 3 $CH_2-CH_2$ | H | 100-103 |
| 185 | " | " | 4 $CH_2CH_2CH_2$ | 3 CO-phenyl | |
| 186 | " | H | " | 3,5 $Cl_2 4CH_3$ | |
| 187 | " | " | " | 4 $NHCOCH_3$ | |
| 188 | " | " | " | 4 $NHCOOC_2H_5$ | |
| 189 | " | " | " | 4 NHCO-phenyl | |
| 190 | " | " | " | 3 $CON(C_2H_5)_2$ | |
| 191 | " | " | " | 3 $SO_2$-phenyl | |
| 192 | " | " | " | 4 $COC_2H_5$ | |
| 193 | " | " | " | 3 $SO_2N(CH_3)_2$ | |
| 194 | " | " | 4 $CH_2CH_2$ | H | 88- 92 |
| 199 | $N(CH_3)_2$ | " | 3 $CH_2CH_2$ | H | |
| 200 | " | " | 4 $CH_2$ | H | |
| 201 | " | " | 4 $CH_2CH_2CH_2CH_2CH_2$ | H | |
| 202 | " | " | 4 $CH-CH_2-CH$ ($CH_3$, $CH_3$) | H | |
| 203 | " | " | 3 $CH_2-C(CH_3)_2CH_2$ | H | |
| 204 | " | " | 4 $CH_2C(CH_3)_2$ $CH_2$ | H | |

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken ; sie sollten in jedem Fall eine feine und gleichmäßige Verteilung der wirksamen Substanzen gewährleisten. Die Formulierungen werden in bekannter Weise hergestellt, z. B. durch Verstrecken des Wirkstoffs mit Lösungsmitteln und/oder Trägerstoffen, ggf. unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen Lösungsmittel in Frage : Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin, Dimethylformamid) und Wasser ; Trägerstoffe wie natürliche Gesteinsmehle (z. B. Kaoline, Tonerden, Talkum, Kreide) und synthetische Gesteinsmehle (z. B. hochdisperse Kieselsäure, Silikate) ; Emulgiermittel wie nichtionogene und anionische Emulgatoren (z. B. Polyoxyethylen — Fettalkohol — Ether, Alkylsulfonate und Arylsulfonate) und Dispergiermittel, wie Lignin, Sulfitablaugen und Methylcellulose.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen oder Gießen. Die Mittel enthalten 0,1 bis 95 % (Gew.-%), vorzugsweise 5 bis 90 % Wirkstoff.

Beispiel I

Man vermischt 90 Gewichtsteile der Verbindung 1 mit 10 Gewichtsteilen N-Methyl-alpha-pyrrolidon und erhält eine Lösung, die zur Anwendung in Form kleinster Tropfen geeignet ist.

Beispiel II

10 Gewichtsteile der Verbindung 2 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

Beispiel III

20 Gewichtsteile der Verbindung 2 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

Beispiel IV

20 Gewichtsteile der Verbindung 3 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht.

Beispiel V

80 Gewichtsteile des Wirkstoffs 1 werden mit 3 Gewichtsteilen des Natriumsalzes der Diisobutylnaphthalin-alphasulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Beispiel VI

5 Gewichtsteile der Verbindung 1 werden mit 95 Gewichtsteilen feinteiligem Kaolin innig vermischt. Man erhält auf diese Weise ein Stäubemittel, das 95 Gewichtsprozent des Wirkstoffs enthält.

Beispiel VII

30 Gewichtsprozent der Verbindung 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel VIII

40 Gewichtsteile des Wirkstoffs 1 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wäßrige Dispersion.

Beispiel IX

20 Teile des Wirkstoffs 1 werden mit 12 Teilen Calciumsalz der Dodecylbenzolsulfonsäure, 8 Teile Fettalkoholpolyglykolether, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

Der Einfluß von Vertretern der neuen Aralkylphenylharnstoffe auf das Wachstum von erwünschten und unerwünschten Pflanzen wird anhand von Gewächshausversuchen vorgeführt:

Als Kulturgefäße dienten Plastikblumentöpfe mit 300 $cm^3$ Inhalt und lehmigem Sand mit etwa 1,5 % Humus als Substrat. Die Samen der Testpflanzen nach Arten getrennt flach eingesät. Unmittelbar danach erfolgte bei Vorauflaufbehandlung das Aufbringen der Wirkstoffe auf die Erdoberfläche. Sie wurden

hierbei in Wasser als Verteilungsmittel suspendiert oder emulgiert und mittels fein verteilender Düsen gespritzt. Bei dieser Applikationmethode handelt es sich um eine Aufwandmenge entsprechend 3,0 kg Wirkstoff/ha. Nach dem Aufbringen der Mittel wurden die Gefäße leicht beregnet, um Keimung und Wachstum in Gang zu bringen. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Wirkstoffe beeinträchtigt wurde. Zum Zwecke der Nachauflaufbehandlung zog man die Testpflanzen je nach Wuchsform erst bis zu einer Wuchshöhe von 3 bis 10 cm an und behandelte sie danach. Zur Nachauflaufbehandlung wurden entweder direkt gesäte und in den gleichen Gefäßen aufgewachsene Pflanzen ausgewählt, oder sie wurden erst als Keimpflanzen getrennt angezogen und einige Tage vor der Behandlung in die Versuchsgefäße verpflanzt. Die Aufwandmenge für die Nachauflaufbehandlung betrug 0,25 kg Wirkstoff/ha. Als Vergleichsmittel diente 1-(3-(2-Phenylethoxy)-phenyl)-3-methoxy-3-methylharnstoff (A) in der gleichen Aufwandmenge. Eine Abdeckung unterblieb bei der Nachauflaufbehandlung. Die Aufstellung der Versuche erfolgte im Gewächshaus, wobei für wärmeliebende Arten wärmere Bereiche (20 bis 30 °C) und für solche gemäßigter Klimate 15 bis 25 °C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Die Ergebnisse zeigen, daß die neue Verbindung Nr. 2 im Gewächshaus bei Nachauflaufbehandlung eine bessere Verträglichkeit für Getreide hat als der bekannte Vergleichswirkstoff (A). Bei Vorauflaufanwendung im Gewächshaus zeigen die Wirkstoffe 4, 14, 38, 47 und 57 eine meistens sehr gute herbizide Wirkung.

Bei Nachauflaufanwendung im Gewächshaus zeigt der Wirkstoff 3 bei Aufwandmengen von 0,25 kg Wirkstoff je ha eine bessere selektive herbizide Wirkung als der bekannte Wirkstoff A. Bei Nachauflaufanwendung im Gewächshaus zeigte der Wirkstoff 16 bei 0,5 kg je ha, der Wirkstoff 100 bei 0,25 kg je ha und der Wirkstoff 22 bei 0,25 kg je ha eine gute selektive herbizide Wirkung.

Bei Nachauflaufanwendung im Gewächshaus zeigten die Wirkstoffe 80, 102, 104, 101 und 109 bei 0,125 und 0,5 kg je ha eine bessere selektive herbizide Wirkung als der bekannte Wirkstoff 1-(3-(2-(2,4-Dichlor)-phenoxyethoxy)-phenyl)-3-methoxy-3-methylharnstoff (D) (DE-OS 28 46 723) bei 0,5 kg je ha.

Sind gewisse Kulturpflanzen gegenüber den Wirkstoffen etwas empfindlich, so können auch Ausbringungstechniken angewandt werden, bei welchen die herbiziden Mittel mit Hilfe der Spritzgeräte so geleitet werden, daß die Blätter empfindlicher Kulturpflanzen nach Möglichkeit nicht getroffen werden, während sie auf die Blätter darunter wachsender unerwünschter Pflanzen oder die unbedeckte Bodenfläche gelangen (post-directed, lay-by). In Anbetracht der guten Verträglichkeit und der Vielseitigkeit der Applikationsmethoden können die erfindungsgemäßen Herbizide noch in einer weiteren großen Zahl von Kulturpflanzen zur Beseitigung unerwünschten Pflanzenwuchses eingesetzt werden.

Die Aufwandmengen können dabei zwischen 0,1 und 15 kg/ha und mehr schwanken.

In Betracht kommen beispielsweise folgende Kulturen :

Tabelle

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fodder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weiße Rübe | turnips |
| Brassica rapa var. silvestris | Rübsen | |
| Camellia sinensis | Teestrauch | tea plants |
| Carthamus tinctorius | Saflor - Färberdistel | safflower |
| Cary illinoinensis | Pekannußbaum | pecan trees |
| Citrus limon | Zitrone | lemon |
| Citrus maxima | Pampelmuse | grapefruits |

Tabelle (Fortsetzung)

| Botanischer Name | Deutscher Name | Englischer Name |
|---|---|---|
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfs and lawn |
| Daucus carota | Möhre | carrots |
| Elaeis guineensis | Ölpalme | oil palms |
| Fragaria vesca | Erdbeere | strawberries |
| Glyoine max | Sojabohne | soybeans |
| Gossypium hirsutum (Gossypium arboreum Gossypium herbaceum Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süßkartoffeln | sweet potato |
| Juglans regia | Walnußbaum | walnut trees |
| Lactua sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |
| Manihot esculenta | Maniok | cassava |
| Medicago sativa | Luzerne | alfalfa (lucerne) |
| Metha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohrkolbenhirse | |
| Petroselinum crispum spp. tuberosum | Wurzelpetersilie | parsley |
| Picea abies | Rotfichte | Norway spruce |
| Abies alba | Weißtanne | fire |
| Pinus spp. | Kiefer | pine trees |

Tabelle (Fortsetzung)

| Botanischer Name | Deutscher Name | Englischer Name |
|---|---|---|
| Pisum sativum | Gartenerbse | English peas |
| Prunus avium | Süßkirsche | cherry trees |
| Prunus domestica | Pflaume | plum trees |
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sasamum indicum | Sesam | Sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preißelbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn maize |

Zur Verbreiterung des Wirkungsspektrums und zur Erzielung synergistischer Effekte können die neuen Aralkylphenylharnstoffe sowohl unter sich als auch mit Zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht. Eine Reihe von Wirkstoffen, welche zusammen mit den neuen Verbindungen für verschiedenste Answendungsbereiche sinnvolle Mischungen ergeben, werden beispielhaft aufgeführt :

α-Naphthoxyessigsäuremethylester
2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy)-acrylat
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
Gibellerinsäure (Salze)
Dinatrium-methylarsonat
Mononatriumsalz der Methylarsonsäure
N-Phosphon-methyl-glycin (Salze)
N,N-Bis-(phosphonmethyl)-glycin (Salze)

2-Chlorethanphosphonsäure-2-chlorethylester
Ammonium-ethyl-carbamoyl-phosphonat
Di-n-butyl-1-n-butylamino-cyclohexyl-phosphonat
Trithiobutylphosphit
O,O-Diisopropyl-5-(2-benzosulfonylamino-ethyl)-phosphordithioat
2,3-Dihydro-5,6-dimethyl-1,4-dithiin-1,1,4,4-tetraoxid 5-tert. Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chlorethyl)-trimethyl-ammoniumchlorid
(2-Methyl-4-phenylsulfonyl)-trifluormethansulfonanilid
1,1-Dimethyl-4,6-diisopropyl-5-indanylethylketon
Natriumchlorat
Ammoniumrhodanid
Calciumcyanamid

5-Amino-4-chlor-2-phenyl-3(2H)-pyridazinon
5-Amino-4-brom-2-phenyl-3(2H)-pyridazinon
5-Amino-4-chlor-2-cyclohexyl-3(2H)-pyridazinon
5-Amino-4-brom-2-cyclohexyl-3(2H)-pyridazinon

5-Methylamino-4-chlor-2-(3-trifluormethylphenyl-3(2H)-pyridazinon
5-Methylamino-4-chlor-2-(3-α,α,β,β-tetrafluorethoxyphenyl)-3(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-cyclohexyl-3(2H)-pyridazinon
4,5-Dimethoxy-2-(3-trifluormethylphenyl)-3(2H)-pyridazinon
5-Methoxy-4-chlor-2-(3-trifluormethylphenyl)-3(2H)-pyridazinon
5-Amino-4-brom-2-(3-methylphenyl)-3(2H)-pyridazinon

3-(1-Methylethyl)-1H-2,1,3-benzothiadiazin-4(3H)-on-2,2-dioxid und Salze
3-(1-Methylethyl)-8-chlor-1H-2,1,3-benzothiadiazin-4(3H)-on-2,2-dioxid und Salze
3-(1-Methylethyl)-8-fluor-1H-2,1,3-benzothiadiazin-4(3H)-on-2,2-dioxid und Salze
3-(1-Methylethyl)-8-methyl-1H-2,1,3-benzothiadiazin-4(3H)-on-2,2-dioxid und Salze

1-Methoxymethyl-3-(1-methylethyl-2,1,3-benzothiadiazin-4(3H)-on-2,2-dioxid
1-Methoxymethyl-8-chlor-3-(1-methylethyl)-2,1,3-benzothiadiazin-4(3H)-on-2,2-dioxid
1-Methoxymethyl-8-fluor-3-(1-methylethyl)-2,1,3-benzothiadiazin-4(3H)-on-2,2-dioxid
1-Cyan-8-chlor-3-(1-methylethyl)-2,1,3-benzothiadiazin-4(3H)-on-2,2-dioxid
1-Cyan-8-fluor-3-(1-methylethyl)-2,1,3-benzothiadiazin-4(3H)-on-2,2-dioxid
1-Cyan-8-methyl-3-(1-methylethyl)-2,1,3-benzothiadiazin-4(3H)-on-2,2-dioxid
1-Cyan-3-(1-methylethyl)-2,1,3-benzothiadiazin-4(3H)-on-2,2-dioxid
1-Azidomethyl-3-(1-methylethyl)-2,1,3-benzothiadiazin-4(3H)-on-2,2-dioxid
3-(1-Methylethyl)-1H-pyridino-[3,2-e]-2,1,3-thiadiazin-(4)-on-2,2-dioxid

N-(1-Ethylpropyl)-2,6-dinitro-3,4-dimethylanilin
N-(1-Methyläthyl)-N-ethyl-2,6-dinitro-4-trifluormethyl-anilin
N-n.Propyl-N-β-chlorethyl-2,6-dinitro-4-trifluormethyl-anilin
N-n.Propyl-N-cyclopropylmethyl-2,6-dinitro-4-trifluormethyl-anilin
N-Bis-(n.propyl)-2,6-dinitro-3-amino-4-trifluormethyl-anilin
N-Bis-(n.propyl)-2,6-dinitro-4-methyl-anilin
N-Bis-(n.propyl)-2,6-dinitro-4-methylsulfonyl-anilin
N-Bis-(n.propyl)-2,6-dinitro-4-aminosulfonyl-anilin
Bis-(β-chlorethyl)-2,6-dinitro-4-methyl-anilin
N-Ethyl-N-(2-methylallyl)-2,6-dinitro-4-trifluormethyl-anilin

N-Methylcarbaminsäure-3,4-dichlorbenzylester
N-Methylcarbaminsäure-2,6-di-tert.butyl-4-methylphenyl-ester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester

N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
3'-N-Isopropyl-carbamoyloxy-propionsäureanilid

Ethyl-N-(3-N'-phenylcarbamoyloxy)-phenyl)-carbamat
Methyl-N-(3-(N'-methyl-N'-phenylcarbamoyloxy)-phenyl)-carbamat
Isopropyl-N-(3-(N'-ethyl-N'-phenylcarbamoyloxy)-phenyl)-carbamat
Methyl-N-(3-(N'-3-methylphenylcarbamoyloxy)-phenyl)-carbamat
Methyl-N-(3-(N'-4-fluorphenylcarbamoyloxy)-phenyl)-carbamat
Methyl-N-(3-(N'-3-chlor-4-fluorphenylcarbamoyloxy)-phenyl)-carbamat
Ethyl-N-(3-(N'-3-chlor-4-fluorphenylcarbamoyloxy)-phenyl)-carbamat
Ethyl-N-(3-(N'-3,4-difluorphenylcarbamoyloxy)-phenyl)-carbamat
Methyl-N-(3-(N'-3,4-difluorphenylcarbamoyloxy)-phenyl)-carbamat

N-3-(4-Fluorphenoxycarbonylamino)-phenylcarbaminsäure-methylester
N-3-(2-Methylphenoxycarbonylamino)-phenylcarbaminsäure-ethylester
N-3-(4-Fluorphenoxycarbonylamino)-phenylthiolcarbaminsäure-methylester
N-3-(2,4,5-Trimethylphenoxycarbonylamino)-phenylthiolcarbaminsäure-methylester
N-3-(Phenoxycarbonylamino)-phenylthiolcarbaminsäuremethyl-ester

N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallyl-ester
N,N-Di-isopropyl-thiolcarbaminsäure-3-methyl-5-isoxazolylmethylester
N,N-Di-isopropyl-thiolcarbaminsäure-3-ethyl-5-isoxazolylmethylester
N,N-Di-sec.butyl-thiolcarbaminsäure-ethylester
N,N-Di-sec.butyl-thiolcarbaminsäure-benzylester
N-Ethyl-N-cyclohexyl-thiolcarbaminsäure-ethylester
N-Ethyl-N-bicyclo-[2,2,1]-heptyl-thiolcarbaminsäure-ethylester
S-(2,3-Dichlorallyl)-(2,2,4-trimethyl-azetidin)-1-carbothiolat
S-(2,3,3-Trichlorallyl)-(2,2,4-trimethyl-azetidin)-1-carbothiolat
S-Ethyl-hexahydro-1-H-azepin-1-carbothiolat
S-Benzyl-3-methylhexahydro-1-H-azepin-1-carbothiolat
S-Benzyl-2,3-dimethylhexahydro-1-H-azepin-1-carbothiolat
S-Ethyl-3-methylhexahydro-1-H-azepin-1-carbothiolat
N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
α,α-Dichlorpropionsäure-Natriumsalz
α,α-Dichlorbuttersäure-Natriumsalz
α,α,β,β-Tetrafluorpropionsäure-Natriumsalz
α-Methyl,α,β-dichlorpropionsäure-Natriumsalz
α-Chlor-β-(4-chlorphenyl)-propionsäure-methylester
α,β-Dichlor-β-phenylpropionsäure-methylester
Benzamido-oxy-essigsäure
2,3,5-Triiodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäuremethylester
2-[4-(2'-Chlor-4'-trifluorphenoxy)-phenoxy]-propionsäure-Natriumsalz
2-[4-(3'-5'-Dichlorpyridyl-2-oxy)-phenoxy]-propionsäure-Natriumsalz

2-(N-Benzoyl-3,4-dichlorphenylamino)-propionsäureethylester
2-(N-Benzoyl-3-chlor-4-fluorphenylamino)-propionsäure-methylester
2-(N-Benzoyl-3-chlor-4-fluorphenylamino)-propionsäure-isopropylester
2-Chlor-4-ethylamino-6-isopropylamino-1,3,5-triazin
2-Chlor-4-ethylamino-6-(amino-2'-propionitril)-1,3,5-triazin
2-Chlor-4-ethylamino-6-2-methoxypropyl-2-amino-1,3,5-triazin
2-Chlor-4-ethylamino-6-butin-1-yl-2-amino-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropoylamino-1,3,5-triazin

2-Azido-4-methylamino-6-isopropylamino-1,3,5-triazin
2-Methylthio-4-ethylamino-6-isopropylamino-1,3,5-triazin
2-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin

2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion

3-tert.Butyl-5-chlor-6-methyluracil
3-tert.Butyl-5-brom-6-methyluracil
3-Isopropyl-5-brom-6-methyluracil
3-sec.Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethyluracil

2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-Allyloxy-1-(4-bromphenyl)-2-«1',2',4'-triazolyl-(1')-]-ethan (Salze)
1-(4-Chlorphenoxy-3,3-dimethyl-1(1H-1,2,4-triazol-1-yl)-2-butanon
N,N-Diallylchloracetamid
N-Isopropyl-2-chloracetanilid
N-(1-Methyl-propin-2-yl)-2-chloracetanilid

2-Methyl-6-ethyl-N-(propargyl)-2-chloracetanilid
2-Methyl-6-ethyl-N-(ethoxymethyl)-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracetanilid
2-Methyl-6-ethyl-N-(4-methoxypyrazol-1-yl-methyl)-2-chloracetanilid
2-Methyl-6-ethyl-N-(pyrazol-1-yl-methyl)-2-chloracetanilid
2,6-Dimethyl-N-(pyrazol-1-yl-methyl)-2-chloracetanilid
2,6-Dimethyl-N-(4-methylpyrazol-1-yl-methyl)-2-chloracetanilid
2,6-Dimethyl-N-(1,2,4-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxolan-2-yl-methyl)-2-chloracetanilid
2,6-Dimethyl-N-(2-methoxyethyl)-2-chloracetanilid
2,6-Dimethyl-N-(isobutoxymethyl)-2-chloracetanilid
2,6-Dimethyl-N-(methoxymethyl)-2-chloracetanilid
2,6-Diethyl-N-(n-butoxymethyl)-2-chloracetanilid
2,6-Diethyl-N-(ethoxycarbonylmethyl)-2-chloracetanilid
2,3,6-Trimethyl-N-(pyrazol-1-yl-methyl)-2-chloracetanilid
2,3-Dimethyl-N-(isopropyl)-2-chloracetanilid
2,6-Diethyl-N-(2-n-propoxyethyl)-2-chloracetanilid

2-(2-Methyl-4-chlorphenoxy)-N-methoxy-acetamid
2-(α-Naphtoxy)-N,N-diethylpropionamid
2,2-Diphenyl-N,N-dimethylacetamid

α-(3,4,5-Tribrompyrazol-1-yl)-N,N-dimethylpropionamid
N-(1,1-Dimethylpropinyl)-3,5-dichlorbenzamid
N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyltrifluormethan-sulfonanilid
5-Acetamido-4-methyl-trifluormethan-sulfonanilid
2-Propionyl-amino-4-methyl-5-chlor-thiazol
O-(Methylsulfonyl)-glykolsäure-N-ethoxymethyl-2,6-dimethylanilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Diiod-4-hydroxy-benzonitril (Salze)
3,5-Dibrom-4-hydroxy-O-2,4-dinitrophenylbenzaldoxim (Salze)
3,5-Dibrom-4-hydroxy-O-2-cyan-4-nitrophenylbenzaldoxim (Salze)
Pentachlorphenol-Natriumsalz
2,4-Dichlorphenyl-4'-nitrophenylether
2,4,6-Trichlorphenyl-4'-nitrophenylether
2-Fluor-4,6-dichlorphenyl-4'-nitrophenylether
2-Chlor-4-trifluormethylphenyl-4'-nitrophenylether
2,4'-Dinitro-4-trifluormethyl-diphenylether
2,4-Dichlorphenyl-3'-methoxy-4'-nitro-phenylether
2-Chlor-4-trifluormethylphenyl-3'-ethoxy-4'-nitro-phenylether
2-Chlor-4-trifluormethylphenyl-3'-carboxy-4'-nitro-phenylether (Salze)
2,4-Dichlorphenyl-3'-methoxycarbonyl-4'-nitro-phenylether
2-(3,4-Dichlorphenyl)-4-methyl-1,2,4-oxadiazolidin-3,5-dion
2-(3-tert.Butylcarbamoyl-oxyphenyl)-4-methyl-1,2,4-oxadiazolidin-3,5-dion
2-(3-iso-Propylcarbamoyl-oxyphenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$0,$^{8,11}$]-dodeca-3,9-dien
2-Ethoxy-2,3-dihydro-3,3-dimethyl-5-benzofuranyl-methan-sulfonat
2-Ethoxy-2,3-dihydro-3,3-dimethyl-5-benzofuranyl-dimethyl-aminosulfonat
2-Ethoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl-allylbernsteinsäureamid
2-Methyl-4,6-dinitrophenol (Salze, Ester)
2-sec.Butyl-4,6-dinitrophenol (Salze)
2-sec.Butyl-4,6-dinitrophenol-acetat
2-tert.Butyl-4,6-dinitrophenol-acetat
2-tert.Butyl-4,6-dinitrophenol (Salze)
2-tert.Butyl-5-methyl-4,6-dinitrophenol (Salze)
2-tert.Butyl-5-methyl-4,6-dinitrophenol-acetat
2-sec.Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-Chlorphenyl)-1-benzoyl-3,3-dimethyl-harnstoff
1-(4-Chlorphenyl)-3,3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(3-α,α,β,β-Tetrafluorethoxyphenyl)-3,3-dimethyl-harnstoff
1-(3-tert.-Butylcarbamoyloxy-phenyl)-3,3-dimethyl-harnstoff

1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4(4'-Chlorphenoxy)-phenyl]-3,3-dimethyl-harnstoff
1-[4(4'-methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethylharnstoff
Imidazolidin-2-on-1-carbonsäure-iso-butylamid
1,2-Dimethyl-3,5-diphenylpyrazolium-methylsulfayt
1,2,4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[(4-methylphenyl)-sulfonyl-oxy]-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1'-Dimethyl-4,4'-dipyridylium-di-(methylsulfat)
1,1'-Di(3,5-dimethylmorpholin-carbonylmethyl)-4,4'-dipyridylium-dichlorid
1,1'-Ethylen-2,2'-dipyridylium-dibromid
3-[1(N-Ethoxyamino)-propyliden]-6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Allyloxyamino)-propyliden]-5,5-dimethylcyclohexan-1,3-dion (Salze)
2-[1-N-Allyloxyamino)-butyliden]-5,5-dimethylcyclohexan-1,3-dion (Salze)
2-[1-(N-Allyloxyamino)-butyliden]-5,5-dimethyl-4-methoxy-carbonyl-cyclohexan-1,3-dion (Salze)
2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)
2-Chlor-4-trifluormethyl-3'-ethoxycarbonyl-4'-nitrophenylether

1-(4-Benzyloxyphenyl)-3-methyl-3-methoxyharnstoff
2-[1-(2,5-Dimethylphenyl)-ethylsulfonyl]-pyridin-N-oxid
1-Acetyl-3-anilino-4-methoxycarbonyl-5-methylpyrazol
3-Anilino-4-methoxycarbonyl-5-methylpyrazol
3-tert.Butylamino-4-methoxycarbonyl-5-methylpyrazol
N-Benzyl-N-isopropyl-trimethylacetamid
2-[4-(4'-Chlorphenoxymethyl)-phenoxy]-propionsäuremethylester
2-[4-(5'-Brompyridyl-2-oxy)-phenoxy]-propionsäureethylester
2-[4-(5'-Jodpyridyl-2-oxy)-phenoxy]-propionsäureethylester
2-[4-(5'-Jodpyridyl-2-oxy)-phenoxy]-propionsäure-n.-butyl-ester
2-Chlor-4-trifluormethylphenyl-3'-(2-fluorethoxy)-4'-nitrophenylether
2-Chlor-4-trifluormethylphenyl-3-(ethoxycarbonyl) methylthio-4-nitrophenylether
2,4,6-Trichlorphenyl-3(ethoxycarbonyl) methylthio-4-nitrophenylether
2-[1-(N-ethoxamino)-butyliden]-5-(2-ethylthiopropyl)-3-hydroxy-cyclohexen-(2)-on-(1) (Salze)
2-[1-(N-ethoxamino)-butyliden]-5-(2-phenylthiopropyl)-3-hydroxy-cyclohexen-(2)-on-(1) (Salze)

4-[4-(4'-Trifluormethyl)-phenoxy]-penten-2-carbonsäure-ethylester
2-Chlor-4-trifluormethyl-3'-methoxycarbonyl-4'-nitrophenylester
2,4-Dichlorphenyl-3'-carboxy-4'-nitrophenylether (Salze)

4,5-Dimethoxy-2-(3-α,α,β-trifluor-β-bromethoxyphenyl)-3-(2H)-pyridazinon
2,4-Dichlorphenyl-3'-ethoxy-ethoxy-ethoxy-4'-nitrophenylether
2,3-Dihydro-3,3-dimethyl-5-benzofuranyl-ethansulfonat
N-[4-Methoxy-6-methyl-1,3,5-triazin-2-yl-aminocarbonyl]-2-chlorbenzolsulfonamid
1(3-Chlor-4-ethoxyphenyl)-3,3-dimethylharnstoff
2-Methyl-4-Chlorphenoxy-thioessigsäureethylester
2-Chlor-3,5-dijod-4-acetoxy-piridin
1(-4-[2-(4-Methylphenyl)-äthoxy]-phenyl-3-methyl-3-methoxy-harnstoff

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

**Ansprüche** (für die Vertragsstaaten : BE, CH, DE, FR, GB, IT, LI, NL, SE)

1. Aralkylphenylharnstoff der Formel

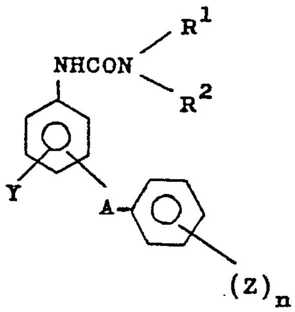

in der

$R^1$ und $R^2$ jeweils unabhängig voneinander Wasserstoff, Methoxy, Ethoxy oder ggf. durch Halogen, Methoxy, Ethoxy oder Cyano substituiertes Alkyl mit 1 bis 4 Kohlenstoffatomen, Cyclopropyl, Cyclohexyl, Allyl, Propargyl oder sekundäres Butinyl oder aber $R^1$ und $R^2$ zusammen eine ggf. durch Methyl oder Ethyl substituierte und/oder ggf. durch Sauerstoff oder Schwefel unterbrochene Alkylenkette mit 2 bis 8 Kohlenstoffatomen,

Y Wasserstoff, Methyl, Tertiärbutyl, Halogen, Methoxy, Ethoxy oder Trifluormethyl,

A einen ggf. durch Methyl oder Ethyl substituierten Alkylenrest mit 1 bis 10 Kohlenstoffatomen,

Z Wasserstoff, Methyl, Ethyl, Tertiärbutyl, Trifluormethyl, Methoxymethyl, Cyclohexyl, Benzyl, Phenyl, Phenoxy, Halogen, eine $C_4H_4$-Kette, die mit dem Benzolring zu einem ggf. substituierten Naphthylring kondensiert ist, Methoxy, Ethoxy, Isopropoxy, Hexyloxy, 1,1,2,2-Tetrafluorethoxy, Methylthio, Thiocyanato, Cyano,

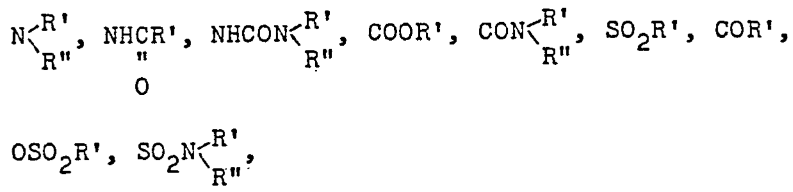

bedeutet, wobei R' und R" jeweils unabhängig voneinander Wasserstoff, Methyl, Ethyl, Methoxy, tert.-Butoxy, Methylthio, Cyclohexyl oder ggf. durch Methyl, Halogen oder Methoxy substituiertes Phenyl bedeuten und n die Zahlen 1 bis 4 bedeutet, ausgenommen diejenigen Verbindungen, in denen A $(CH_2)_2$ und gleichzeitig $R^1$ und $R^2$ Wasserstoff und Z Wasserstoff bedeutet, sowie derjenigen Verbindung, in denen A $(CH_2)_3$ oder $(CH_2)_4$ und gleichzeitig $R^1$ Methyl, $R^2$ Methyl oder Methoxy, Y Wasserstoff oder Chlor, Z Wasserstoff oder $C_1$-$C_4$-Alkyl und n = 1 bedeutet.

2. Herbizid, enthaltend einen Aralkylphenylharnstoff gemäß Anspruch 1.

3. Herbizid, enthaltend einen festen oder flüssigen Trägerstoff und einen Aralkylphenylharnstoff gemäß Anspruch 1.

4. Verfahren zur Herstellung eines Herbizids, dadurch gekennzeichnet, daß man einen festen oder flüssigen Trägerstoff vermischt mit einem Aralkylphenylharnstoff gemäß Anspruch 1.

5. Verfahren zur Bekämpfung von unerwünschten Pflanzen, dadurch gekennzeichnet, daß man die Pflanzen oder den Boden behandelt mit einem Aralkylphenylharnstoff gemäß Anspruch 1.

6. Verfahren zur Herstellung eines Aralkylphenylharnstoffs gemäß Anspruch 1, dadurch gekennzeichnet, daß man ein Isocyanat der Formel

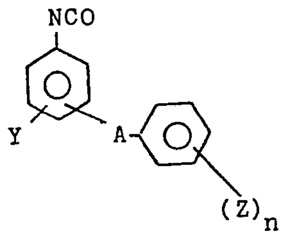

in der A, Y, Z und n die obengenannten Bedeutungen haben, mit einem Amin der Formel

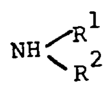

in der $R^1$ und $R^2$ die obengenannten Bedeutungen haben, umsetzt.

**Ansprüche** (für den Vertragsstaat AT)

1. Herbizid, enthaltend einen Aralkylphenylharnstoff der Formel

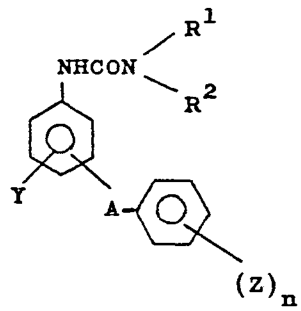

in der

$R^1$ und $R^2$ jeweils unabhängig voneinander Wasserstoff, Methoxy, Ethoxy oder ggf. durch Halogen, Methoxy, Ethoxy oder Cyano substituiertes Alkyl mit 1 bis 4 Kohlenstoffatomen, Cyclopropyl, Cyclohexyl, Allyl, Propargyl oder sekundäres Butinyl oder aber $R^1$ und $R^2$ zusammen eine ggf. durch Methyl oder Ethyl substituierte und/oder ggf. durch Sauerstoff oder Schwefel unterbrochene Alkylenkette mit 2 bis 8 Kohlenstoffatomen,

Y Wasserstoff, Methyl, Tertiärbutyl, Halogen, Methoxy, Ethoxy oder Triffluormethyl,

A einen ggf. durch Methyl oder Ethyl substituierten Alkylenrest mit 1 bis 10 Kohlenstoffatomen,

Z Wasserstoff, Methyl, Ethyl, Tertiärbutyl, Trifluormethyl, Methoxymethyl, Cyclohexyl, Benzyl, Phenyl, Phenoxy, Halogen, eine $C_4H_4$-Kette, die mit dem Benzolring zu einem ggf. substituierten Naphthylring kondensiert ist, Methoxy, Ethoxy, Isopropoxy, Hexyloxy, 1,1,2,2-Tetrafluorethoxy, Methylthio, Thiocyanato, Cyano,

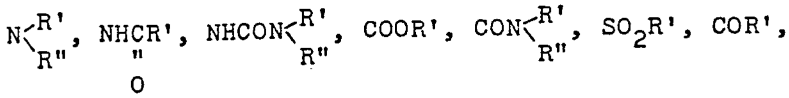

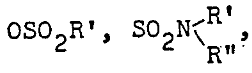

bedeutet, wobei R' und R'' jeweils unabhängig voneinander Wasserstoff, Methyl, Ethyl, Methoxy, tert.-Butoxy, Methylthio, Cyclohexyl oder ggf. durch Methyl, Halogen oder Methoxy substituiertes Phenyl

bedeuten und n die Zahlen 1 bis 4 bedeutet, ausgenommen diejenigen Verbindungen, in denen A $(CH_2)_2$ und gleichzeitig $R^1$ und $R^2$ Wasserstoff und Z Wasserstoff bedeutet.

2. Herbizid, enthaltend einen festen oder flüssigen Trägerstoff und einen Aralkylphenylharnstoff der Formel

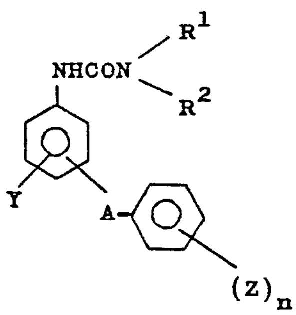

in der

$R^1$ und $R^2$ jeweils unabhängig voneinander Wasserstoff, Methoxy, Ethoxy oder ggf. durch Halogen, Methoxy, Ethoxy oder Cyano substituiertes Alkyl mit 1 bis 4 Kohlenstoffatomen, Cyclopropyl, Cyclohexyl, Allyl, Propargyl oder sekundäres Butinyl oder aber $R^1$ und $R^2$ zusammen eine ggf. durch Methyl oder Ethyl substituiertes und/oder ggf. durch Sauerstoff oder Schwefel unterbrochene Alkylenkette mit 2 bis 8 Kohlenstoffatomen,

Y Wasserstoff, Methyl, Tertiärbutyl, Halogen, Methoxy, Ethoxy oder Trifluormethyl,

A einen ggf. durch Methyl oder Ethyl substituierten Alkylenrest mit 1 bis 10 Kohlenstoffatomen,

Z Wasserstoff, Methyl, Ethyl, Tertiärbutyl, Trifluormethyl, Methoxymethyl, Cyclohexyl, Benzyl, Phenyl, Phenoxy, Halogen, eine $C_4H_4$-Kette, die mit dem Benzolring zu einem ggf. substituierten Naphthylring kondensiert ist, Methoxy, Ethoxy, Isopropoxy, Hexyloxy, 1,1,2,2-Tetrafluorethoxy, Methylthio, Thiocyanato, Cyano,

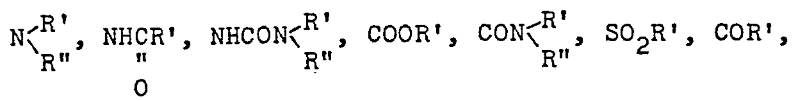

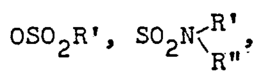

bedeutet, wobei R' und R'' jeweils unabhängig voneinander Wasserstoff, Methyl, Ethyl, Methoxy, tert.-Butoxy, Methylthio, Cyclohexyl oder ggf. durch Methyl, Halogen oder Methoxy substituiertes Phenyl bedeuten und n die Zahlen 1 bis 4 bedeutet.

3. Verfahren zur Herstellung eines Herbizids, dadurch gekennzeichnet, daß man einen festen oder flüssigen Trägerstoff vermischt mit einem Aralkylphenylharnstoff definiert wie in Anspruch 2.

4. Verfahren zur Bekämpfung von unerwünschten Pflanzen, dadurch gekennzeichnet, daß man die Pflanzen oder den Boden behandelt mit einem Aralkylphenylharnstoff definiert wie in Anspruch 2.

5. Verfahren zur Herstellung eines Aralkylphenylharnstoffs definiert wie in Anspruch 1, dadurch gekennzeichnet, daß man ein Isocyanat der Formel

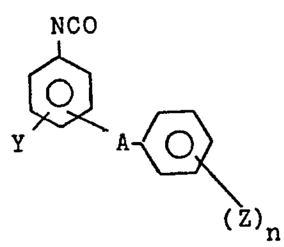

in der A, Y, Z und n die obengenannten Bedeutungen haben, mit einem Amin der Formel

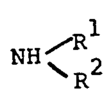

in der $R^1$ und $R^2$ die obengenannten Bedeutungen haben, umsetzt.

**Claims** (for the Contracting States : BE, CH, DE, FR, GB, IT, LI, NL, SE)

1. An aralkylphenylurea of the formula

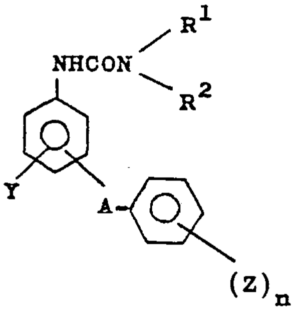

where

$R^1$ and $R^2$ are each hydrogen, methoxy, ethoxy or unsubstituted or halogen-, methoxy-, ethoxy-, or cyano-substituted alkyl of 1 to 4 carbon atoms, cyclopropyl, cyclohexyl, allyl, propargyl or sec.-butynyl, or $R^1$ and $R^2$ together are an unsubstituted or methyl- or ethyl-substituted alkylene chain of 2 to 8 carbon atoms which may or may not be interrupted by oxygen or sulfur,

Y is hydrogen, methyl, tert.-butyl, halogen, methoxy, ethoxy or trifluoromethyl,

A is unsubstituted or methyl- or ethyl-substituted alkylene of 1 to 10 carbon atoms,

Z is hydrogen, methyl, ethyl, tert.-butyl, trifluoromethyl, methoxymethyl, cyclohexyl, benzyl, phenyl, phenoxy, halogen, a $C_4H_4$ chain which is fused to the benzene ring to give a substituted or unsubstituted naphthyl ring, methoxy, ethoxy, isoproxy, hexyloxy, 1,1,2,2-tetrafluoroethoxy, methylthio, thiocyanato, cyano,

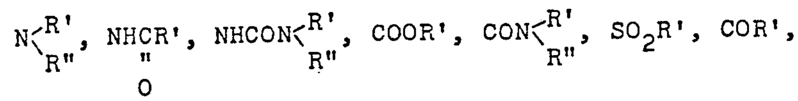

$OSO_2R'$, or 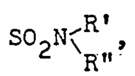

R′ and R″ each being hydrogen, methyl, ethyl, methoxy, tert.-butoxy, methylthio, cyclohexyl or unsubstituted or methyl-, halogen- or methoxy-substituted phenyl, and n is 1, 2, 3 or 4, excluding those compounds in which A is $(CH_2)_2$ and, at the same time, $R^1$, $R^2$ and Z are each hydrogen, and those compounds in which A is $(CH_2)_3$ or $(CH_2)_4$ and, at the same time, $R^1$ is methyl or methoxy, Y is hydrogen or chlorine, Z is hydrogen or $C_1$-$C_4$-alkyl, and n is 1.

2. A herbicide containing an aralkylphenylurea as claimed in claim 1.

3. A herbicide containing a solid or liquid carrier and an aralkylphenylurea as claimed in claim 1.

4. A process for producing a herbicide, wherein a solid or liquid carrier is mixed with an aralkylphenylurea as claimed in claim 1.

5. A process for combating unwanted plants, wherein the plants or the soil are treated with an aralkylphenylurea as claimed in claim 1.

6. A process for preparing an aralkylphenylurea as claimed in claim 1, wherein an isocyanate of the formula

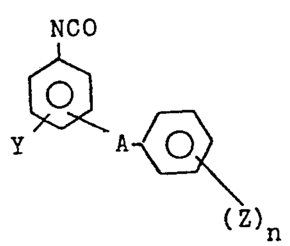

where A, Y, Z and n have the above meanings, is reacted with an amine of the formula

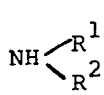

where $R^1$ and R2 have the above meanings.

**Claims** (for the Contracting State AT)

1. A herbicide containing an aralkylphenylurea of the formula

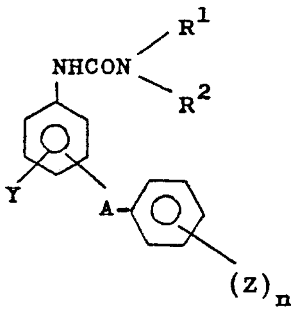

where

$R^1$ and $R^2$ are each hydrogen, methoxy, ethoxy or unsubstituted or halogen-, methoxy-, ethoxy- or cyano-substituted alkyl of 1 to 4 carbon atoms, cyclopropyl, cyclohexyl, allyl, propargyl or sec.-butynyl, or $R^1$ and $R^2$ together are an unsubstituted or methyl- or ethyl-substituted alkylene chain of 2 to 8 carbon atoms which may or may not be interrupted by oxygen or sulfur.

Y is hydrogen, methyl, tert.-butyl, halogen, methoxy, ethoxy or trifluoromethyl,

A is unsubstituted or methyl- or ethyl-substituted alkylene of 1 to 10 carbon atoms,

Z is hydrogen, methyl, ethyl, tert.-butyl, trifluoromethyl, methoxymethyl, cyclohexyl, benzyl, phenyl, phenoxy, halogen, a $C_4H_4$ chain which is fused to the benzene ring to give a substituted or unsubstituted naphthyl ring, methoxy, ethoxy, isopropoxy, hexyloxy, 1,1,2,2-tetrafluoroethoxy, methylthio, thiocyanato, cyano,

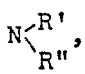, 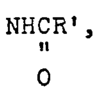, 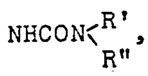, COOR', 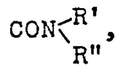, $SO_2R'$, COR',

$OSO_2R'$, or 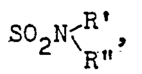,

R' and R" each being hydrogen, methyl, ethyl, methoxy, tert.-butoxy, methylthio, cyclohexyl or unsubstituted or methyl-, halogen- or methoxysubstituted phenyl, and n is 1, 2, 3 or 4, excluding those compounds in which A is $(CH_2)_2$ and, at the same time, $R^1$, $R^2$ and Z are each hydrogen.

2. A herbicide containing a solid or liquid carrier and an aralkylphenylurea of the formula

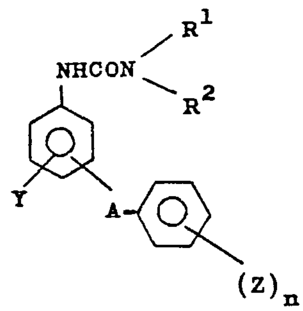

where

$R^1$ and $R^2$ are each hydrogen, methoxy, ethoxy or unsubstituted or halogen-, methoxy, ethoxy or cyano-substituted alkyl of 1 to 4 carbon atoms, cyclopropyl, cyclohexyl, allyl, propargyl or sec.-butynyl, or $R^1$ and $R^2$ together are an unsubstituted or methyl- or ethyl-substituted alkylene chain of 2 to 8 carbon atoms which may or may not be interrupted by oxygen or sulfur,

Y is hydrogen, methyl, tert.-butyl, halogen, methoxy, ethoxy or trifluoromethyl,

A is unsubstituted or methyl- or ethyl-substituted alkylene of 1 to 10 carbon atoms,

Z is hydrogen, methyl, ethyl, tert.-butyl, trifluoromethyl, methoxymethyl, cyclohexyl, benzyl, phenyl, phenoxy, halogen, a $C_4H_4$ chain which is fused to the benzene ring to give a substituted or unsubstituted naphthyl ring, methoxy, ethoxy, isopropoxy, hexyloxy, 1,1,2,2-tetrafluoroethoxy, methylthio, thiocyanato, cyano,

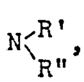, 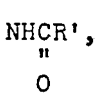, 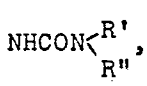, COOR', 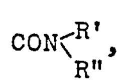, $SO_2R'$, COR',

$OSO_2R'$, or 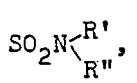,

R′ and R″ each being hydrogen, methyl, ethyl, methoxy, tert.-butoxy, methylthio, cyclohexyl or unsubstituted or methyl-, halogen- or methoxy-substituted phenyl, and n is 1, 2, 3 or 4.

3. A process for producing a herbicide, wherein a solid or liquid carrier is mixed with an aralkylphenylurea as defined in claim 2.

4. A process for combating unwanted plants, wherein the plants or the soil are treated with an aralkylphenylurea as defined in claim 2.

5. A process for preparing an aralkylphenylurea as defined in claim 1, wherein an isocyanate of the formula

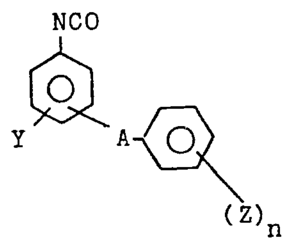

where A, Y, Z and n have the above meanings, is reacted with an amine of the formula

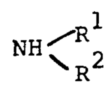

where $R^1$ and $R^2$ have the above meanings.

**Revendications** (pour les Etats contractants : BE, CH, DE, FR, GB, IT, LI, NL, SE)

1. Aralkylphénylurée de formule

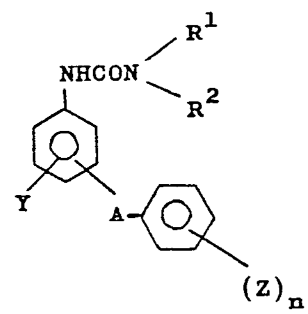

dans laquelle

$R^1$ et $R^2$ représentent, chacun, indépendamment l'un de l'autre, hydrogène, méthoxy, éthoxy, ou alkyle à 1 à 4 atomes de carbone, éventuellement substitué par halogène, méthoxy, éthoxy ou cyano, cyclopropyle, cyclohexyle, allyle, propargyle ou butinyle secondaire, ou bien $R^1$ et $R^2$ ensemble une chaîne alkylène de 2 à 8 atomes de carbone, éventuellement substituée par méthyle ou éthyle et/ou éventuellement interrompue par oxygène ou soufre,

Y représente hydrogène, méthyle, tert.butyle, halogène, méthoxy, éthoxy ou trifluorométhyle,

A un reste alkylène de 1 à 10 atomes de carbone éventuellement substitué par méthyle ou éthyle,

Z représente hydrogène, méthyle, tert.butyle, trifluorométhyle, méthoxyméthyle, cyclohexyle, benzyle, phényle, phénoxy, halogène, une chaîne $—C_4H_4$ qui, avec le noyau benzène est condensée en un noyau naphtyle éventuellement substitué, méthoxy, éthoxy, isopropoxy, hexyloxy, 1,1,2,2-tétrafluoréthoxy, méthylthio, thiocyanato, cyano

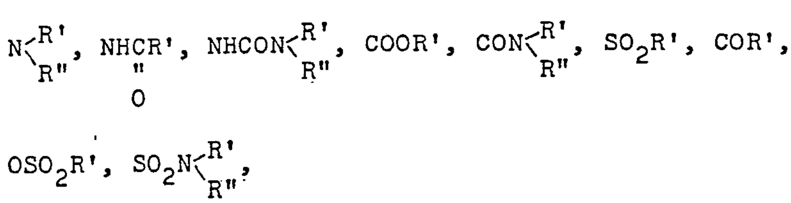

R′ et R″ représentant chacun, indépendamment l'un de l'autre, hydrogène, méthyle, éthyle, méthoxy, tert.butoxy, méthylthio, cyclohexyle, ou phényle, éventuellement substitué par méthyle, halogène, ou méthoxy et n les nombres 1 à 4, à l'exclusion des composés dans lesquels A représente

$(CH_2)_2$ et simultanément $R^1$ et $R^2$ représentent hydrogène et Z représente hydrogène, ainsi que des composés dans lesquels A représente $(CH_2)_3$ ou $(CH_2)_4$ et simultanément $R^1$ représente méthyle, $R^2$ méthyle ou méthoxy, Y hydrogène ou chlore, Z hydrogène ou alkyle en $C_1$-$C_4$ et n = 1.

2. Herbicide contenant une aralkylphénylurée selon la revendication 1.

3. Herbicide contenant un support solide ou liquide et une aralkylphénylurée selon la revendication 1.

4. Procédé pour préparer un herbicide, caractérisé par le fait qu'on mélange un support solide ou liquide avec une aralkylphénylurée selon la revendication 1.

5. Procédé de lutte contre les plantes indésirables, caractérisé par le fait qu'on traite les plantes ou le sol avec une aralkylphénylurée selon la revendication 1.

6. Procédé de préparation d'une aralkylphénylurée selon la revendication 1, caractérisé par le fait qu'on fait réagir un isocyanate de formule

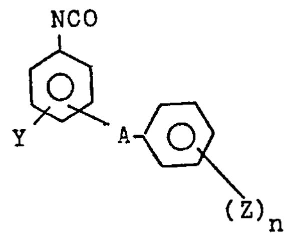

dans laquelle A, Y, Z et n ont les significations sus-indiquées, avec une amine de formule

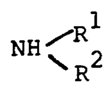

dans laquelle $R^1$ et $R^2$ ont les significations sus-mentionnées.

**Revendications** (pour l'Etat contractant AT)

1. Herbicide contenant une aralkylphénylurée de formule

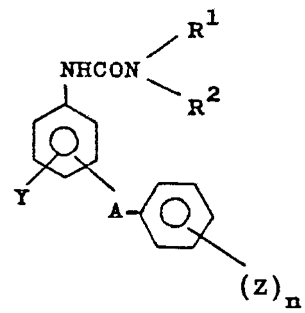

dans laquelle

$R^1$ et $R^2$ représentent, chacun, indépendamment l'un de l'autre, hydrogène, méthoxy, éthoxy, ou alkyle à 1 ou 4 atomes de carbone, éventuellement substitué par halogène, méthoxy, éthoxy ou cyano, cyclopropyle, cyclohexyle, allyle, propargyle ou butinyle secondaire, ou bien $R^1$ et $R^2$ ensemble une chaîne alkylène de 2 à 8 atomes de carbone, éventuellement substituée par méthyle ou éthyle et/ou éventuellement interrompu par oxygène ou soufre,

Y représente hydrogène, méthyle, tert.butyle, halogène, méthoxy, éthoxy ou trifluorométhyle,

A un reste alkylène de 1 à 10 atomes de carbone éventuellement substitué par méthyle ou éthyle,

Z représente hydrogène, méthyle, tert.butyle, trifluorométhyle, méthoxyméthyle, cyclohexyle, benzyle, phényle, phénoxy, halogène, une chaîne $-C_4H_4$ qui, avec le noyau benzène est condensée en un noyau naphtyle, éventuellement substitué, méthoxy, éthoxy, isopropoxy, hexyloxy 1,1,2,2-tétrafluoréthoxy, méthylthio, thiocyanate, cyano,

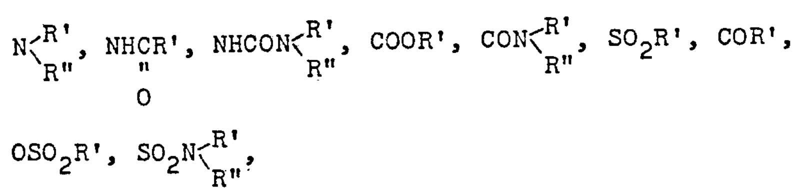

R' et R" représentent chacun, indépendamment l'un de l'autre, hydrogène, méthyle, éthyle, méthoxy, tert.butoxy, méthylthio, cyclohexyle, ou phényle, éventuellement substitué par méthyle,

halogène, ou méthoxy et n les nombres 1 à 4, à l'exclusion des composés dans lesquels A représente $(CH_2)_2$ et simultanément $R^1$ et $R^2$ représentent hydrogène et Z représente hydrogène.

2. Herbicide contenant un support solide ou liquide et une aralkylphénylurée de formule

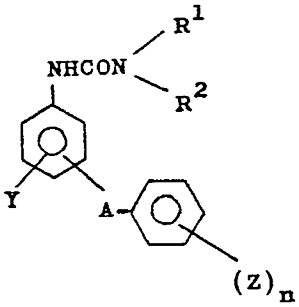

dans laquelle

$R^1$ et $R^2$ représentent, chacun, indépendamment l'un de l'autre, hydrogène, méthoxy, éthoxy, ou alkyke à 1 à 4 atomes de carbone, éventuellement substitué par halogène, méthoxy, éthoxy ou cyano, cyclopropyle, cyclohexyle, allyle, propargyle ou butinyle secondaire, ou bien $R^1$ et $R^2$ ensemble une chaîne alkylène de 2 à 8 atomes de carbone, éventuellement substituée par méthyle ou éthyle et/ou éventuellement interrompue par oxygène ou soufre,

Y représente hydrogène, méthyle, tert.butyle, halogène, méthoxy, éthoxy ou trifluorométhyle,

A un reste alkylène de 1 à 10 atomes de carbone éventuellement substitué par méthyle ou éthyle,

Z représente hydrogène, méthyle, tert.butyle, trifluorométhyle, méthoxyméthyle, cyclohexyle, benzyle, phényle, phénoxy, halogène, une chaîne $—C_4H_4$ qui, avec le noyau benzène est condensée en un noyau naphtyle éventuellement substitué, méthoxy, éthoxy, isopropoxy, hexyloxy, 1,1,2,2-tétrafluoréthoxy, méthylthio, thiocyanato, cyano

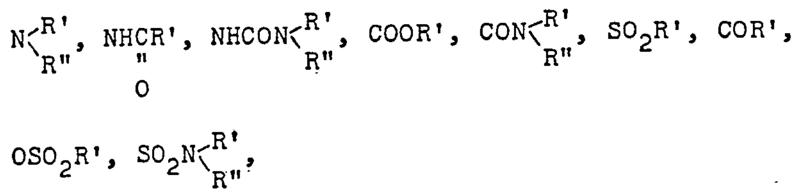

R' et R" représentent chacun, indépendamment l'un de l'autre, hydrogène, méthyle, éthyle, méthoxy, tert.butoxy, méthylthio, cyclohexyle, ou phényle, éventuellement substitué par méthyle, halogène, ou méthoxy et n les nombres 1 à 4.

3. Procédé pour préparer un herbicide, caractérisé par le fait qu'on mélange un support solide ou liquide avec une aralkylphénylurée définie comme dans la revendication 2.

4. Procédé de lutte contre les plantes indésirables, caractérisé par le fait qu'on traite les plantes ou le sol avec une aralkylphénylurée définie comme dans la revendication 2.

5. Procédé de préparation d'une aralkylphénylurée définie comme dans la revendication 1, caractérisé par le fait qu'on fait réagir un isocyanate de formule

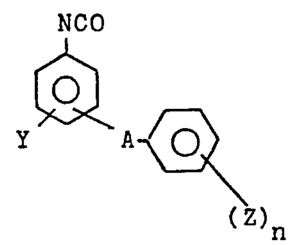

dans laquelle A, Y, Z, et n ont les significations sus-indiquées, avec une amine de formule

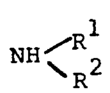

dans laquelle $R^1$ et $R^2$ ont les sigifications sus-mentionnées.